(19) Europäisches Patentamt

European Patent Office

Office européen des brevets

(11) **EP 1 306 030 B1**

(12) **EUROPEAN PATENT SPECIFICATION**

(45) Date of publication and mention
of the grant of the patent:
**10.05.2006 Bulletin 2006/19**

(51) Int Cl.:
**A45D 44/00** *(2006.01)* **A45D 33/38** *(2006.01)*
**D21H 17/67** *(2006.01)*

(21) Application number: **01954411.3**

(22) Date of filing: **01.08.2001**

(86) International application number:
**PCT/JP2001/006631**

(87) International publication number:
**WO 2002/011580 (14.02.2002 Gazette 2002/07)**

(54) **COSMETIC PAPER**

KOSMETIKPAPIER

PAPIER COSM TIQUE

(84) Designated Contracting States:
**DE FR GB IT**

(30) Priority: **02.08.2000 JP 2000234908**

(43) Date of publication of application:
**02.05.2003 Bulletin 2003/18**

(73) Proprietor: **SHISEIDO COMPANY, LTD.**
**Chuo-ku,**
**Tokyo 104-8010 (JP)**

(72) Inventors:
• **SADAOKA, Toshihiro**
**c/o Daio Paper Corporation**
**Iyomishima-shi,**
**Ehime 799-0492 (JP)**
• **KATSUBE, Hiroaki**
**Iyomishima-shi,**
**Ehime 799-0492 (JP)**
• **TAKECHI, Yuji**
**Iyomishima-shi,**
**Ehime 799-0492 (JP)**
• **TAKEDA, Akira**
**Iyomishima-shi,**
**Ehime 799-0492 (JP)**
• **MATSUMOTO, Yoshio**
**Nishinomiya-shi, Hyogo 663-8113 (JP)**
• **MATSUMOTO, Katsuji**
**Toyonaka-shi, Osaka 560-0052 (JP)**

(74) Representative: **Held, Stephan et al**
**Meissner, Bolte & Partner GbR**
**Postfach 86 03 29**
**81630 München (DE)**

(56) References cited:
**EP-A2- 0 393 723** **WO-A1-92/14781**
**WO-A1-97/40817** **JP-A- 11 137 336**

**Description**

Field of the Invention

The invention relates to a sebum absorbing paper containing inorganic filler comprising hydroxyapatite.

Background of the Invention

On a human's face, the skin surface, in particular around the nose and chin and at the middle of the forehead, tends to be oily due to the frequent secretion of sebum. Makeup on such oily skin surface avoids a cosmetic product such as a moisture retaining agent or toilet powder from being applied properly to the skin surface, resulting in decreased makeup-effect. Additionally, the human skin is damaged by the oxidized sebum generated by its exposure to the air while the time passes after its secretion.

In the prior art, among the various kinds of cosmetic tissues, the sebum absorbing papers, which are thin and soft and excel in lipid absorbency, were particularly used for removing the sebum. But, in these days, beside such sebum absorbing papers, there are even other kinds of cosmetic tissues, which are also thin and soft and excel in lipid absorbency. Generally, when each cosmetic tissue for removing sebum absorbs the sebum, this tissue's transparency increases at the parts where the sebum is absorbed, whereby a user can recognize visually the effect of lipid absorbency.

The sebum forms film on the skin surface so as to prevent transepidermal water loss. Therefore, it is not desirable, from the viewpoint of skin care, that the sebum is removed excessively. Actually, the conventional cosmetic tissues, particularly sebum absorbing papers may remove the sebum excessively. Precisely, they may absorb not only old sebum, which became acid due to oxidation while the time passes after its secretion, but also new sebum, which keeps freshness soon after its secretion and would be required for retaining the moisture of skin. The oxidized sebum is harmful to user's skin, thus, a sebum absorbing paper is required to absorb such sebum selectively among the several kinds of sebum so that the damage to the user's skin can be decreased.

The document EP 0 393 723 A discloses absorbing papers.

Disclosure of the Invention

It is therefore, the main object of the present invention is to provide a sebum absorbing paper, which excels in the absorbency of the oxidized sebum selectively among the several kinds of sebum.

According to the present invention, there is provided a sebum absorbing paper used for applying to skin or for cleaning the skin, which contains 1 to 30 weight % of inorganic filler and which has the basis weight of 5 to 25 g/m$^2$ as defined in JIS P-8124, wherein the above inorganic filler comprises hydroxyapatite.

Since this sebum absorbing paper contains 1 to 30 weight % of inorganic filler and has the basis weight of 5 to 25 g/m$^2$ as defined in JIS P-8124, this paper can be thin and soft so as to be used comfortably.

Since the hydroxyapatite adsorbs efficiently the oxidized sebum selectively among the various kinds of sebum, this paper contains the inorganic filler comprising at least 1 to 100 weight % of hydroxyapatite so that this paper can absorb efficiently the oxidized sebum.

It is preferable that this paper has the density of 0.4 to 1 g/m$^3$ as defined in JIS P-8118. It is also preferable that the average particle size of the above inorganic filler is 0.5 to 8 $\mu$m. Further, it is more preferable that this paper contains the inorganic filler, which comprises 0.5 to 90 weight % of talc as well as the hydroxyapatite. If the average particle size of the inorganic filler is smaller than 0.5 $\mu$m, the stability of the filler in the paper will be decreased, resulting in disadvantageous cost effectiveness and small removability of the lipid. On the other hand, if the average particle size of the inorganic filler is larger than 8 m, some problems will be caused, precisely, the texture of the paper will be much lowered and the particles will be easily pulled off from the paper. When this paper contains the inorganic filler comprising 0.5 to 90 weight % of talc, the smoothness and thereby the texture of this paper will be improved. In addition, thanking to the talc contained in this paper, it can become easily and surely transparent when it absorbs the lipid.

Then, it is preferable that pulverized talc has the average particle size of 0.5 to 2 $\mu$m. Due to the talc having such particle size, the smoothness and thereby the texture of the paper will be improved.

This paper may comprise moisture retaining agent and/or toilet powder. Due to those comprised, the paper further excels in skin care-effect and makeup-effect.

Brief Description of the Drawing

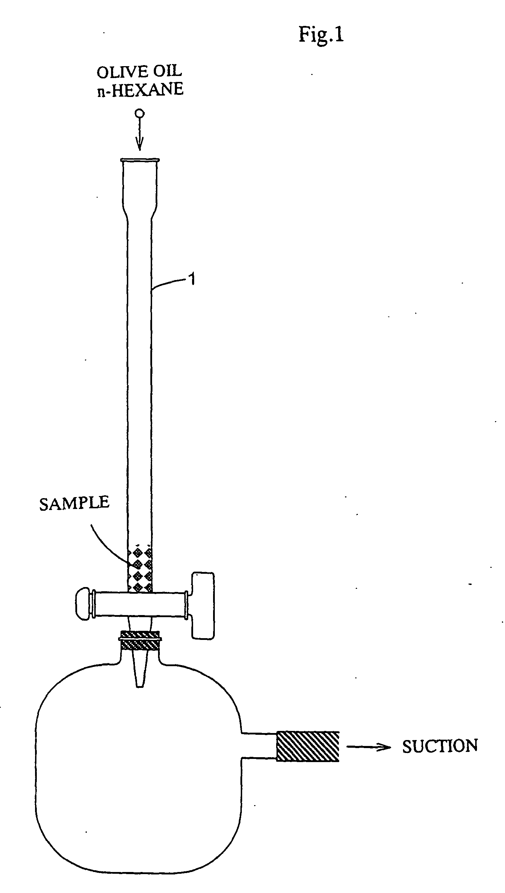

Fig. 1 is a schematic illustration of apparatus used for measuring the adsorptivity for lipid peroxide.

Best Mode for Carrying Out the Invention

Now, the present invention will be described more closely, referring to the following embodiments.

The hydroxyapatite [$Ca_{10}(PO_4)_6(OH)_2$] is included in calcium phosphate and known as a component of a human bone or tooth. Then, the hydroxyapatite has attracted interest as an industrial material, for example, a material having sufficient adsorptivity for positive ion, negative ion, protein, amino acid, and the like, and a material having biological compatibility due to its small effect on organism, e.g. as the material of an artificial tooth root.

The hydroxyapatite excels in adsorption of lipid peroxide selectively among several kinds of lipid. Thus, this hydroxyapatite can adsorb oxidized sebum selectively among the several kinds of sebum generated by secretion on the skin. The present inventors considered such property of the hydroxyapatite and attained its application to a cosmetic tissue. Consequently, there is provided a sebum absorbing paper, which contains 1 to 30 weight % of inorganic filler and which has the basis weight of 5 to 25 $g/m^2$ as defined in JIS P-8124, wherein the above inorganic filler comprises 1 to 100 weight % of hydroxyapatite. The resultant paper is thin and soft and the oxidized sebum occupies high proportion in all sebum absorbed into this paper. In this specification, the sebum absorbing paper is a cosmetic tissue which is applied to skin for absorbing the sebum or which cleans the skin by absorbing the sebum.

In the present invention, there are various ways for introducing the inorganic filler into the sebum absorbing paper. Concretely, the filler is originally added into the paper, or the inorganic filler containing the hydroxyapatite is coated on a base paper, which has been produced through a paper machine. If the amount of inorganic filler contained in the sebum absorbing paper exceeds 30 weight %, the paper will exhibit hardness, which means insufficient softness. On the other hand, if the amount of hydroxyapatite contained in the above inorganic filler is smaller than 1 weight %, this paper will not absorb the oxidized sebum, which leads to ineffectiveness.

The sebum absorbing paper in the present invention can be produced in a known method with a known paper machine. For example, the sebum absorbing paper can be produced through a known paper machine from fibrous pulp slurry, which contains the inorganic filler comprising the hydroxyapatite.

In the present invention, as the fiber used for the material of the sebum absorbing paper, the following materials are listed. There are wood pulp fiber, bast fiber such as Manila hemp, flax, hemp, jute, paper mulberry, paper bush, and ganpi, bast fiber such as cotton, straw, bamboo, and kenaf, chemical fiber such as acrylic fiber and rayon, and animal fiber such as silk. It is possible that one of these fibers is solely used as the material. Then, it is also possible that two or more than two of these fibers are combined and used. In the present invention, since the sebum absorbing paper produced through a paper machine has the basis weight of 5 to 25 $g/m^2$, thin and soft paper can be attained. If the paper has the basis weight of smaller than 5 $g/m^2$, the mechanical strength of this paper will be lowered, resulting in that the paper will be twisted and torn easily in use. On the other hand, if the basis weight exceeds 25 $g/m^2$, it will take much cost to produce this paper and the softness of this paper will be decreased, which leads to uncomfortable use.

It is desirable that, in the sebum absorbing paper in accordance with the present invention, the amount of absorbed lipid equals or exceeds 1 $g/m^2$ as defined in the following measuring method for the amount of pseudo absorbed lipid. If the amount of absorbed lipid is smaller than 1 $g/m^2$, the paper will not suitable because of too small absorptivity. On the other hand, when the amount of absorbed lipid equals or exceeds 1 $g/m^2$, it can be said that this paper may absorb the sufficient amount of the sebum. That is to say, in this sebum absorbing paper, contained hydroxyapatite fulfills the function for adsorbing the oxidized sebum selectively among the various kinds of sebum.

Now, the above measuring method for the amount of pseudo absorbed sebum will be stated. First, a sample sheet having the dimension of 21 cm x 25 cm is fixed by means of adhesive tape or the like on the circumferential face of a printability tester's drum so that the sample sheet has the effective area of 19 cm x 18 cm. Next, 0.5 ml of oil liquid (80 weight % of caster oil and 20 weight % of benzyl alcohol) is coated uniformly on a printer roller of the above printability tester so that the thickness of the coated oil liquid is 4.8 $\mu$m. Then, the printer roller is turned fully once at the transfer revolution speed of 30 rpm with the nip of 5 mm between the above drum and printer roller so that the above oil liquid can be transferred to the above sample sheet. Weight increase of the sample sheet having the effective area due to the transfer is calculated by subtracting its weight before the transfer from its weight after the transfer. On the basis of this weight increase, the amount of absorbed lipid per 1 $m^2$ of sample sheet can be obtained as shown in the following equation.

$$\text{amount of absorbed lipid (g/ m}^2)$$

$$= \text{weight of sample sheet having effective area after transfer (g) / effective area } (\text{m}^2)$$

$$- \text{weight of sample sheet having effective area before transfer (g) / effective area } (\text{m}^2)$$

In the cosmetic tissue used for absorbing and removing sebum like typically the sebum absorbing paper, when it absorbs the sebum, its transparency increases at the parts where the sebum is absorbed, whereby a user can visually recognize the removal of the sebum. In order to estimate such increase of the transparency, the present inventors use the value for punch force. The punch force can be calculated from color difference of sample paper caused by the absorbing of the lipid. As the transparency on the absorbing of the lipid is increased, value for the punch force is also increased, which gives satisfaction to the user.

According to the present invention, the above punch force of the sebum absorbing paper preferably equals or exceeds 5.0. The paper having such punch force shows, on the absorbing of the lipid, the increase in the transparency, whereby the user can recognize visually the effect of the lipid absorbency. Now, the measuring method of the punch force will be stated. The back face of a sample paper is covered with a black plate, then with a white plate. The values Lw, Aw, and Bw, then the values Lb, Ab, and Bb of the paper, respectively, are measured before transfer with a light spectrophotometer "EPR-80WX" (manufactured by Tokyo Denshoku Kabushikigaisha). As for the sample paper before the transfer, the color difference $\Delta$ E1 between these two cases is calculated according to the following equation (4). Next, transfer is carried out in the same manner as the above measurement of the amount of absorbed sebum. Precisely, a sample sheet having the dimension of 21 cm x 25 cm is fixed by means of adhesive tape or the like on the circumferential face of a printability tester's drum so that the sample sheet has the effective area of 19 cm x 18 cm. Consequently, 0.5 ml of oil liquid (80 weight % of caster oil and 20 weight % of benzyl alcohol) is coated uniformly on a printer roller of the above printability tester so that the thickness of the coated oil liquid is 4.8 $\mu$m. After this, the printer roller is turned fully once at the transfer revolution speed of 30 rpm with the nip of 5 mm between the above drum and printer roller so that the above oil liquid can be transferred to the above sample sheet. Then, as for the sample paper after the transfer, the color difference $\Delta$ E2 between these two cases is calculated according to the above equation (2) in the same manner as the sample paper before the transfer. Finally, the punch force $\Delta$ E can be obtained from the color difference of the sample paper before the transfer $\Delta$ E1 and the color difference of the sample paper after the transfer $\Delta$ E2, according to the following equation (3).

$$\Delta \text{ En} = \{(\text{Lw - Lb})^2 + (\text{Aw - Ab})^2 + (\text{Bw - Bb})^2\}^{1/2}$$

where Lw represents the value of paper covered with the white plate, Lb represents the value of paper covered with the black plate, Aw represents the blue to yellow-hue of the paper covered with the white plate, Ab represents the blue to yellow-hue of the paper covered with the black plate, Bw represents the red to green-hue of the paper covered with the white plate, and Bb represents the red to green-hue of the paper covered with the black plate.

$$\Delta \text{ E} = \Delta \text{ E1} + \Delta \text{ E2}$$

where $\Delta$ E represents the punch force.

It is preferable that the inorganic filler contained in the sebum absorbing paper in accordance with the present invention comprises 0.5 to 90 weight % of talc as well as the hydroxyapatite. The average particle size of the talc is 0.5 to 8 $\mu$m. It is more preferable that pulverized talc has the average particle size of 0.5 to 2 $\mu$m. The average particle size of the talc can be measured by a known device Microtrack. Graded talc, which is available from market, e.g. SG-2000 manufactured by Nihon Talc Kabushikigaisha can be used. The talc contained in the paper contributes the smoothness and thereby the texture of this paper, resulting in great comfortable use.

The sebum absorbing paper surpasses in the texture and in the absorbency of the oxidized sebum. Generally, in a cosmetic tissue, particularly in the sebum absorbing paper, when it absorbs the sebum, its transparency increases at the parts where the sebum is absorbed. In this connection, the sebum absorbing paper in accordance with the present invention excels in improvement of the transparency, whereby the user can recognize visually and clearly the effect of the sebum absorbency, which leads to user's satisfaction.

The sebum absorbing paper according to the present invention may contain moisture retaining agent, toilet powder and so on. As the moisture retaining agent, a material having such function can be used. For example, there are hohoba oil, glycerol, 1,3-butylene glycol, hyaluronic acid, collagen and the like.

The sebum absorbing paper of the present invention can be colored with a coloring agent. By coloring the sebum absorbing paper, contrast between the parts where the sebum is absorbed and the parts where the sebum is not absorbed is shown clearly, which further makes easy for the user to recognize the effect of the sebum absorbency. As the coloring agent, known dye like basic dye, acid dye, direct dye or the like and known pigment can be used. In the case that the pigment is used, the paper is neither blurred nor faded with the pigment. When the dye is used, food dye is preferably used, because the paper is brought into direct contact with the skin.

[Example]

As for the sebum absorbing paper of the present invention, its punch force, amount of absorbed lipid, adsorptivity for lipid peroxide, durability in use and productivity through a paper machine were tested at the different proportions of components in its inorganic filler. The results are shown in the following Table. The method for measuring the punch force and the amount of absorbed lipid were already stated above. The adsorptivity for the lipid peroxide was measured in the following. First, 1 g of sample was introduced into a column 1 of apparatus configured as shown in Fig. 1. Next, 2 g of olive oil (manufactured by Wako Pure Chemical Industries Ltd.) was diluted with 4 g of n-hexane. Then, thus obtained olive oil was fed downwardly from the top of the column 1 under reduced pressure. Consequently, 100 ml of n-hexane was fed downwardly from the top of the column 1 so that effluent was obtained. Thus obtained effluent was evaporated in a water bath so as to be reduced to 10 ml. 10 ml of remained effluent was removed from the water bath. Then, 10 ml of glacial acetic acid and 2 g of potassium iodide were added into this effluent while this mixture was stirred. Titration was carried out for the free iodine in this mixture with 0.01 mol/l of sodium thiosulfate in a buret. The end point was detected with a starch indicator. From the result of this titration, peroxide value could be obtained. Finally, the amount of adsorbed lipid peroxide could be calculated from the obtained peroxide value.

Table

| sample No | basis weight | filler | proportion (wt %) | | | | | punchforce | absorbed lipid | adsorptivity for L.P. | durability in use | productivity through P.M. | general estimation |
|---|---|---|---|---|---|---|---|---|---|---|---|---|---|
| | | | HA | talc | α-alumina | silica | sericite | | | | | | |
| | g/m² | wt % | | (P.S.) μm | (P.S.) μm | (P.S.) μm | (P.S.) μm | Δ E | g/m² | % | | | |
| 1 | 25 | 30 | 100 (5.0) | - | - | - | - | ○ | ◎ | ◎ | ○ | ○ | ◎ |
| 2 | 15 | 15 | 50 (8) | 50 (0.5) | - | - | - | ◎ | ◎ | ◎ | ◎ | ○ | ◎ |
| 3 | 5 | 5 | 10 (0.5) | 90 (5.0) | - | - | - | ◎ | ○ | ○ | ◎ | ○ | ◎ |
| 4 | 15 | 15 | - | 100 (2.0) | - | - | - | ◎ | ◎ | × | ◎ | ○ | × |
| 5 | 15 | 10 | 50 (6.0) | 50 (8.0) | - | - | - | ○ | ○ | ◎ | ○ | ○ | ○ |
| 6 | 15 | 15 | - | - | 100 (1.0) | - | - | × | ○ | × | × | × | × |
| 7 | 15 | 15 | 95 (8.0) | - | 5 (2.0) | - | - | ○ | ○ | ○ | ○ | ○ | ○ |
| 8 | 15 | 15 | - | - | - | 100 (5.0) | - | × | ◎ | × | × | ○ | × |
| 9 | 15 | 15 | 95 (8.0) | - | - | 5 (3.5) | - | ○ | ○ | ○ | ○ | ○ | ○ |
| 10 | 15 | 15 | - | - | - | - | 100 (5.0) | × | ○ | × | × | × | × |
| 11 | 15 | 15 | 95 (8.0) | - | - | - | 5 (6.5) | ○ | ○ | ○ | ○ | ○ | ○ |

| sample No | basis weight | filler | proportion (wt %) | | | | | punchforce | absorbed lipid | adsorptivity for L.P. | durability in use | productivity through P.M. | general estimation |
|---|---|---|---|---|---|---|---|---|---|---|---|---|---|
| | | | HA | talc | α-alumina | silica | sericite | | | | | | |
| | g/m$^2$ | wt % | | (P.S.) μm | (P.S.) μm | (P.S.) μm | (P.S.) μm | Δ E | g/m$^2$ | % | | | |
| 12 | 4 | 15 | 100 (8.0) | - | - | - | - | ○ | ○ | ◎ | × | × | × |
| 13 | 28 | 15 | 100 (8.0) | - | - | - | - | ○ | ◎ | ◎ | × | ○ | × |
| 14 | 15 | 15 | 50 (8.0) | 50 (10.0) | - | - | - | ○ | ○ | ◎ | × | ○ | × |
| 15 | 15 | 15 | 99.5 (8.0) | 0.5 (8.0) | - | - | - | ○ | ◎ | ◎ | ○ | ○ | ◎ |

For the punch force, mark × means "smaller than 5", mark ○ means " exceeds 5 but is smaller than 15", and mark ◎ means " equals or exceeds 15".

For the absorbed lipid, mark × means "smaller than 1.0", mark ○ means " exceeds 1.0 but is smaller than 2.0", and mark ◎ means " equals or exceeds 2.0".

For the adsorptivity for lipid peroxide, mark × means "smaller than 5", mark ○ means " exceeds 5 but is smaller than 20", and mark ◎ means " equals or exceeds 20".

For the durability in use, in trials by 50 users, mark × means "this paper is twisted and torn" and mark ○ means "this paper is neither twisted nor torn".

For the productivity through paper machine, in producing each paper, mark × means "problems such as paper break, dehydration failure and the like are caused" and mark ○ means "any problems are not caused".

For the general estimation, mark ○ means "the effect of the present invention can be attained, in other words, the lipid peroxide can be adsorbed", mark ◎ means "taking account of the smoothness, amount of absorbed lipid and punch force, this paper can be suitably used, particularly as the sebum absorbing paper".

The sebum absorbing paper, which has the basis weight and inorganic filler as defined in the present invention, is thin and soft and has enough durability in use for the application to a cosmetic tissue, particularly to a sebum absorbing paper.

The sample containing no hydroxyapatite does not have the effect of the present invention, i.e., adsorptivity for the lipid peroxide. Then, it is confirmed that the sample comprising talc and/or hydroxyapatite has the preferable punch force. In addition, it is recognized that the sebum absorbing paper in accordance with the present invention has no problem as for the productivity through a paper machine.

As a result, from the above example, the sebum absorbing paper in accordance with the present invention is thin and soft and excels in the absorbency of the oxidized lipid. Further, it should be noted that this sebum absorbing paper preferably contains talc in order to serve as a cosmetic tissue, particularly as a sebum absorbing paper.

[Experiments]

Several experiments were carried out in order to confirm the hydroxyapatite's adsorptivity for the sebum. The hydroxyapatite was obtained by synthesis through a wet process. Then, the obtained material was dehydrated centrifugally and dried for 24 hours at the temperature of 70 °C. The resultant hydroxyapatite was sized so as to have the mesh size of 400 or smaller, whereby the sample was finally obtained. As comparative materials of the inorganic filler, $\alpha$-alumina, sericite, talc and silica were used. As the pseudo sebum, three materials, i.e., oleic acid, oleic oxide and olive oil were used respectively.

The amount of adsorbed lipid, which had been derived from each pseudo-sebum, was quantitatively determined by a thermal analysis. Then, it was confirmed that the hydroxyapatite adsorbed the lipid advantageously over the sericite and over the $\alpha$-alumina. The olive oil was fed through a column packed with the hydroxyapatite and the peroxide value of the olive oil was measured. As a result, the peroxide value was decreased, which means that the hydroxyapatite has the adsorptivity for the lipid peroxide. The samples were applied on a cheek of an experimental subject. Then, for each sample, the amount of adsorbed sebum was measured by a thermal analysis. As a result, it is found that the hydroxyapatite has the largest adsorptivity for the sebum and for the oxidized sebum.

It is clear from the foregoing description that according to the present invention, there is provided a sebum absorbing paper, which is thin and soft, which can remove efficiently the acid sebum selectively among the various kinds of sebum and which excels in skin-care.

**Claims**

1.    A sebum absorbing paper used for applying to skin or for cleaning the skin, comprising:

     1 to 30 % by weight inorganic filler, said inorganic filler comprises 1 to 100 % by weight hydroxyapatite, wherein the paper has a basis weight of 5 to 25 g/m$^2$ and is capable of absorbing selectively oxidized sebum.

2.    A sebum absorbing paper according to claim 1, wherein an average particle size of said inorganic filler is 0,5 to 8 $\mu$m.

3.    A sebum absorbing paper according to claim 1 or 2, wherein said inorganic filler comprises 0,5 to 90 % by weight talc having the average particle size of 0,5 to 8 $\mu$m.

**Patentansprüche**

1.    Sebum absorbierendes Papier, das zum Auftragen auf Haut oder zur Reinigung der Haut verwendet wird, umfassend: 1 bis 30 Gew.-% anorganischen Füllstoff, wobei der genannte anorganische Füllstoff 1 bis 100 Gew.-% Hydroxyapatit umfasst, wobei das Papier ein Basisgewicht von 5 bis 25 g/m$^2$ hat und fähig ist, oxidiertes Sebum selektiv zu absorbieren.

2.    Sebum absorbierendes Papier nach Anspruch 1, wobei eine durchschnittliche Partikelgröße des anorganischen Füllstoffs 0,5 bis 8 $\mu$m ist.

3.    Sebum absorbierendes Papier nach Anspruch 1 oder 2, wobei der genannte anorganische Füllstoff 0,5 bis 90 Gew.-% Talk mit einer durchschnittlichen Partikelgröße von 0,5 bis 8 $\mu$m umfasst.

**Revendications**

1.  Papier absorbant le sébum, utilisé pour une application à la peau ou pour un nettoyage de la peau, comprenant :

    1 à 30 % en poids d'une charge minérale, ladite charge minérale comprend 1 à 100 % en poids d'hydroxyapatite, dans lequel
    le papier a un grammage de 5 à 25 g/m$^2$ et est capable d'absorber sélectivement le sébum oxydé.

2.  Papier absorbant le sébum selon la revendication 1, dans lequel une taille moyenne de particules de ladite charge minérale est de 0,5 à 8 $\mu$m.

3.  Papier absorbant le sébum selon la revendication 1 ou 2, dans lequel ladite charge minérale comprend 0,5 à 90 % en poids de talc ayant une taille moyenne de particules de 0,5 à 8 $\mu$m.

Fig.1

OLIVE OIL
n-HEXANE

1

SAMPLE

SUCTION